# EUROPEAN PATENT APPLICATION

(11) **EP 2 669 009 A1**
(43) Date of publication of application: **04.12.2013**
(21) Application number: 12170144.5
(22) Date of filing: 31.05.2012
(51) Int. Cl.: B01J 31/06, B01J 31/02, B01J 37/34, D06M 14/32, D06M 14/34

(54) **Process for the immobilization of catalysts on textile materials, the obtained textile materials and the use of said materials**

(71) Applicant: Studiengesellschaft Kohle mbH, 45470 Mülheim (DE)
(72) Inventor: List, Benjamin, 45470 Mülheim an der Ruhr (DE); Lee, Ji-Wong, 45470 Mülheim an der Ruhr (DE); Mayer-Gall, Thomas, 47475 Kamp-Lintfort (DE); Opwis, Klaus, 47623 Kevelaer (DE); Gutmann, Jochen Stefan, 47803 Krefeld (DE)

(57) **Abstract**

The present invention relates to methods for the preparation of solid-supported organic catalysts, particularly on textile materials via photochemical immobilization. More specifically, the present invention relates to the organocatalysis and recognition process by using the textile-supported chiral molecules.

## Description

The present invention relates to processes for the preparation of solid-supported organic catalysts, particularly on textile materials via photochemical immobilization. Furthermore, the present invention relates to processes making use of the textile-supported catalysts, in particular in the synthesis of chiral molecules using organocatalysis.

Catalysts lower the activation energy of chemical reactions compared to the non-catalyzed reaction. This reduces the energy requirement of a reaction drastically, and enables the environmentally friendly and economical chemical production of a variety of valuable compounds. Practically, most of well-known chemical processes employ heterogeneous catalysts for easy separation of the product and convenient recycling of the catalyst. Also, heterogeneous catalysts can easily be applied to continuous flow process. Moreover, it is desirable to establish methodology for the preparation of heterogeneous variants of homogeneous catalysts via simple and practical modification procedures.

Heterogeneous catalysis can be divided into two groups:
- Immobilized metals and metal oxides with large and active surface area, for example, a catalytic converter which converts toxic chemicals to less toxic ones for most of the vehicles, the Haber process for ammonia synthesis' and the Fischer-Tropsch process for the conversion of carbon monoxide and hydrogen into liquid hydrocarbons as an example of a heterogeneous transition metal catalyzed process. 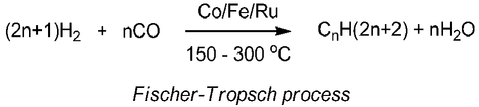
- Immobilized organic molecules with an active site for:
   1) transition metal catalysis as a ligand
   2) biocatalysis using high-molecular-weight enzymes, and
   3) organocatalysis, especially using chiral organocatalysts for asymmetric syntheses.

During the last dozen years, organocatalysis emerged as a major field of asymmetric synthesis complementing biocatalysis and transition metal catalysis. Its versatility has been established in numerous organic transformations with unprecedented activity and high selectivity. By employing small organic molecules, the reactions could be catalyzed via covalent activation, hydrogen bonding interaction and ionic interactions. To achieve reasonable reaction outcome with high selectivity, it is often required to use a fairly large catalyst loading which may hamper an industrial application of organocatalysis.

To overcome above mentioned obstacles and to realize the possibility of recycling the organic catalyst, a number of strategies have been explored for preparing heterogeneous organocatalysts via covalent and non-covalent immobilization. For example, the Jacobsen group utilized Merrifield's resin for the preparation of a library of thiourea catalysts for asymmetric Strecker synthesis. Extensive studies were conducted for immobilization of secondary amine catalysts via co-polymerization, click chemistry, ionic liquid formation. Also, functionalization of alkene functional group can give a facile access for heterogeneous organocatalyst. For example, hydrosilylation of cinchona alkaloid catalyzed by platinum catalyst (i. e. Speier's and Karstedt's) can generate silylated compounds, which can be grafted onto a heterogeneous surface.

All the described methods have significant drawbacks. First of all, the methods, using polystyrene based resin (i.e. Merrifield resin, Wang resin, JandaJel™) for immobilization, require complicated modifications of the monomeric catalyst or the resin itself to install an appropriate functional group. In this process, unreacted functional groups remain on the surface and will affect or interrupt the catalysis. Moreover, additional functional groups on the catalyst may alter the activity and selectivity of the heterogenized catalyst versus its homogenous counterpart. Furthermore, such modification processes often require multiple organic transformations, which ultimately prevent industrial applications.

Recently, co-polymerization of monomeric catalysts and self-supported catalysts ("bottom-up" strategy) to produce heterogeneous organocatalysts are emerging as an alternative pathway. Cowley's group has reported the catalysis with a heterogeneous imidazolium salt as a carbene precursor. This orthogonally positioned heterogenous carbene catalyst was used for three cycles without great loss of reactivity. However, this methodology also requires complicated pre-modification of the catalyst monomer. Oxidative polymerization of thiophene functional group can directly generate porous heterogeneous organocatalyst without sophisticated modification of catalyst monomer. However, since the thiophene substituent can alter the activity and selectivity of the catalysis, stereochemical outcome of the heterogeneous catalysis was inferior to homogenous catalysis with bulky substituents.

To solve this limitation, it is a great challenge to find versatile solid material which is robust and abundant for large scale application. As already mentioned, the advantage of heterogeneous catalysis is that no separation of the catalyst is required and that the catalyst can be recycled without sophisticated purification. In accordance with this ultimate goal, the morphology of the heterogeneous material has to be easy to handle for various applications. To address this problem, various catalytic resins were prepared with the bead form not displaying powder morphology. However, this requires additional engineering technology for their preparation. Also, for high performance of the heterogeneous catalyst, it is highly desirable to obtain high mass transfer efficiency to the active sites and prevent catalyst leach-out during the catalysis, which requires a permanent chemical connection between catalyst and solid support. Also, the total porosity and the pore distribution have an important role. These parameters directly influence the catalyst loading capacity of the material, the material conversion to the corresponding immobilized catalyst, and the separation ability of the overall system of the reaction matrix.

Concerning all these parameters, heterogeneous catalysts that are immobilized in various ways are relatively expensive and not applicable for large scale production. Therefore, it is highly desirable to develop an efficient immobilization technique, which can be applied to the industrial use of asymmetric organocatalysts.

The inventors found out that a substrate-specific immobilization method can provide permanently immobilized catalysts on polymeric materials such as textile surfaces via a photo-induced cross-linking reaction. To provide a general solution for the described problems, the presented invention is related to the facile immobilization of organocatalysis on a suitable textile material via simple one-step photochemical process. After covalent immobilization, heterogeneous organocatalysts can not be leached-out during the reaction and/or recycling process. Leaching-out problem is often observed in heterogeneous organometallic catalysis, which will eventually contaminate the reaction product with transition metal impurities.

The solution of the problem underlying the invention is achieved by providing a general method for an immobilization of organic catalysts on the polymeric carrier materials, specifically, textile materials. The immobilization process is conducted by using UV light irradiation of a solid material and subsequent quenching with an appropriate organic molecule, which is immobilized on the solid surface photochemically.

The invention is therefore directed to a process for immobilization of at least one organic catalyst on a polymeric support, wherein said organic catalyst is permanently bonded to the polymeric support by a chemical interaction between at least one functional group on either of the organic catalyst and the polymeric support.

The chemical interaction is generally a chemical bonding via a ionic or radically induced reaction, and can be induced by a wet chemical process or a photochemical process.

The organic catalyst is preferably a heterogeneous organic catalyst, preferably selected from any of cinchona alkaloid-based bifunctional catalysts, BINOL-based phosphoric acids, BINOL-based imidodiphosphoric acids, secondary- and primary amine catalysts, nitrogen-based nucleophilic catalysts, TEMPO as an organic oxidant, phase-transfer catalysts as well as imidodiphosphoric acids as disclosed in EP application No.12150663.8 of the same applicant. The catalyst is particularly a chiral heterogeneous organic catalyst.

The polymeric support is advantageously selected from the group of natural or synthetic textile materials or mixtures thereof, preferably selected from polyesters, polyamides, polyacrylates, polyolefins, cotton, rayon and wool.

Though the interaction is preferably induced by way of a photochemically induced process such as irradiation with UV light, it is also possible to induce said immobilization via a wet chemical process.

As preferred measure, the organic catalyst is immobilized on the polymeric support via a photochemical reaction between at least one functional crosslinking olefinic group on the organic catalyst with the polymeric support, either directly or via a linker molecule having at least two vinylic or allylic olefinic groups such as PETA or TAC.

The functional crosslinking olefinic group is advantageously a vinyl or allyl group attached to the organic catalyst, and the photochemical reaction is induced by irradiation with UV light, in particular with UV light of a wavelength in the range of 100 to 350 nm, preferably with a wavelength of 222 nm.

In one embodiment, the polymeric support is treated or impregnated with a solution of the organic catalyst having at least one functional crosslinking olefinic group in a photochemically inert organic solvent, the obtained impregnated polymeric support is irradiated with UV light and the obtained polymeric support having the organic catalyst covalently fixed thereon is recovered and optionally washed with an organic solvent. Photochemically inert organic solvent means that the solvent Is capable of dissolving the used catalyst, but does not take part in the photochemical reaction or interaction between the catalyst moiety and the polymeric support. Leading to the catalyst immobilized on the polymeric support.

Thus, the invention provides a simple and rapid method for permanent immobilization of organic catalysts on polymeric textiles. Various catalysts can be immobilized and used several times without loss of activity. Particularly, textile materials provide a solid substrate by the good permeability for organic solvent and show excellent substrate conversion to the desired product with high selectivity. In addition, the flexibility of solid materials allows a practical use in various types of reactors without sophisticated packing or reaction set-ups.

Polymeric textile materials have been used since the pre-historic periods because of their high accessibility and numerous advantageous functionalities. One can use basically any textile materials, for example, those from vegetable, animal and insects. However, synthetic textile materials such as polyesters, polyamides, polyacrylates or polyolefins are much cheaper than styrene-based resins and highly durable under various conditions and easily accessible.

Moreover, due to the flexible textile structure it is highly applicable to diverse reactors with any geometry. In addition, textile fabrics and immobilized catalyst can be removed quickly and without leaving any residue from a reactor. The structure of low-bonded non-woven products ensures a large specific surface area and high permeability. Also textile materials can potentially render high catalyst loading and rapid mass transfer and thus it enables high turnover number and frequency. Finally, various textile materials with different chemical backbones render a variety of choices for immobilization process to produce comparable environment effect as homogeneous catalysis.

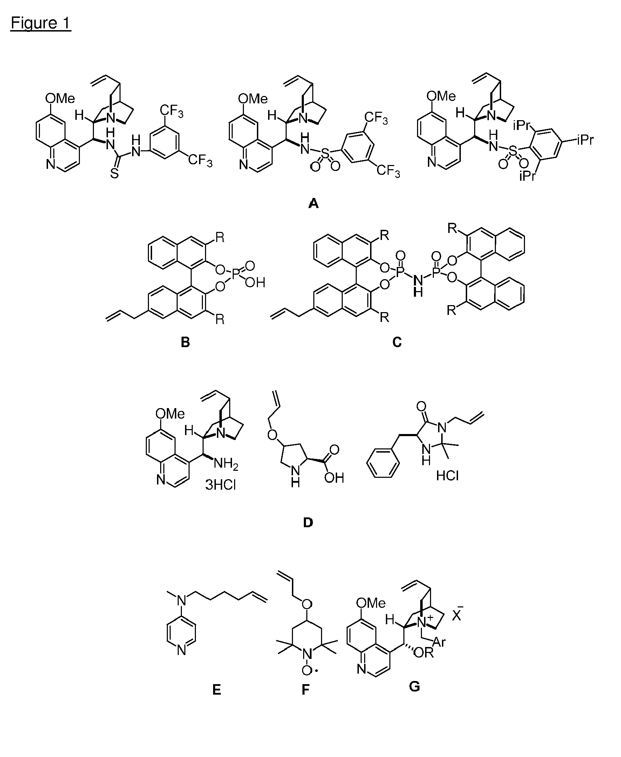

For the purpose of the present invention, various organocatalysts, which show high catalytic efficiency for valuable organic transformations, were immobilized on textile materials. This organocatalyst can be, for example, any of cinchona alkaloid-based bifunctional catalysts (A), BINOL-based phosphoric acids (B), BINOL-based imidodiphosphoric acids (C) including those, secondary- and primary amine catalysts (D), nitrogen-based nucleophilic catalysts (E), TEMPO as an organic oxidant (F) and phase-transfer catalysts (G) in Figure 1 and those imidodiphosphoric acids as disclosed in EP application No.12150663.8. All the catalysts are preferably functionalized with an olefin functional group for the photochemical immobilization.

The invention relates to methods of preparation of organocatalyst-supported materials and the use of the same, in particular for catalysis in chemical reactions, more specifically, asymmetric organic reactions. All the methods make use of an organocatalyst having at least one olefin functional group for the photochemical immobilization on the textile carrier, preferably selected from any of polyesters, polyamides, polyacrylates or polyolefins or copolymers thereof. The immobilization can take place via a photochemical immobilization step or via a wet chemical immobilization step.

First, the inventive photochemical immobilization can generally proceed as follows: The organic catalyst functionalized with at least one carbon-carbon double bond is dissolved in a suitable solvent. A polymeric support material is wetted with the solution. Subsequently, the polymeric carrier material is irradiated with UV light. By the irradiation, the organic compound is permanently fixed to the support material. For the photochemical immobilization, UV light has an appropriate wavelength that initiates the grafting reaction according to the invention, for example, to bind the olefin group to the textile materials, UV light with a wavelength ranging from 100 nm to 350 nm can be used. If it is necessary, to increase catalyst loading, catalytic activity and selectivity cross linker can be used. Such linker can provide two or more binding sites for one organocatalyst per binding site, respectively, each organocatalyst having at least one olefin functional group, so that more than one, at least two organocatalysts can be bonded to the carrier via said linker.

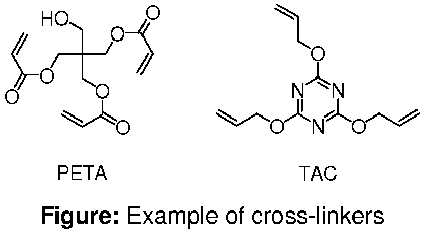

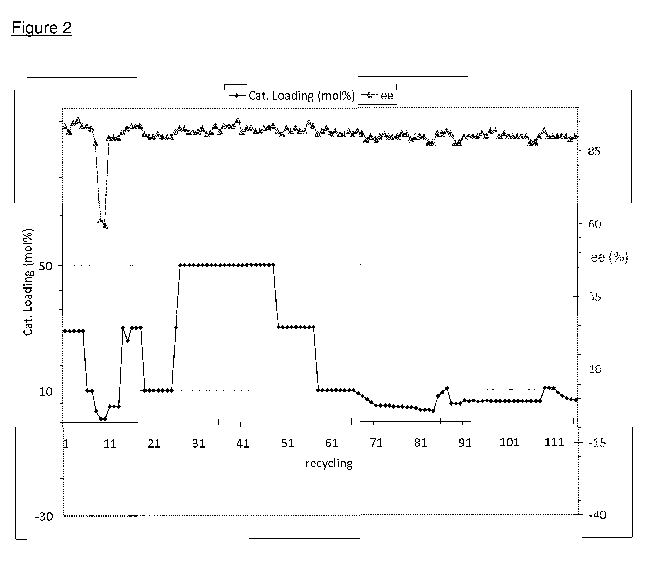

The invention is further illustrated by the following examples and attached drawings. In said drawings, Figure 1 shows some illustrative embodiments of the catalysts which can be immobilized on the polymeric support according to the invention, said catalysts having structural formulae which can be further modified for controlling the catalytic activity of the catalyst. Figure 2 shows the selectivity vs. the number of reaction cycles of one embodiment of the inventive material.

### Example 1:

The photochemical immobilization of quinine and its derivatives is generally described in the following scheme.

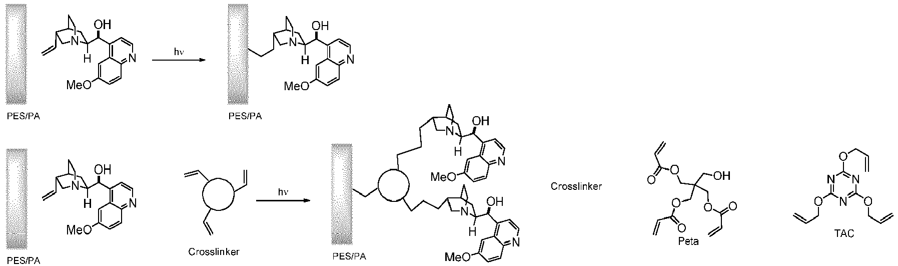

The catalytic activity and enantioselectivity are as follows:

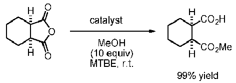

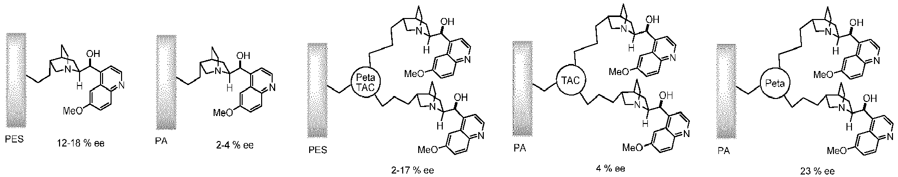

Recyclability of quinine-immobilized textile catalyst is as follows:

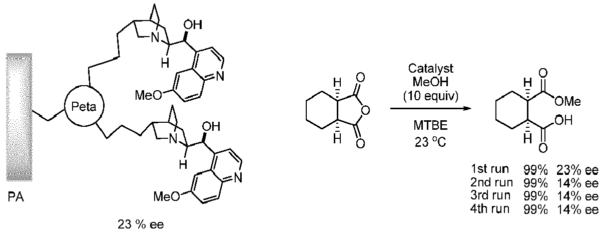

The quinine-immobilized textiles were used for desymmetrization of meso-anhydride and gave catalytic reactivity but enantioselectivities were inferior. However, through the recycling experiments, enantioselectivity was maintained even though ee values were low.

### Example 2:

The photochemical immobilization of cinchona alkaloids and its derivatives is generally shown in the following schemes:

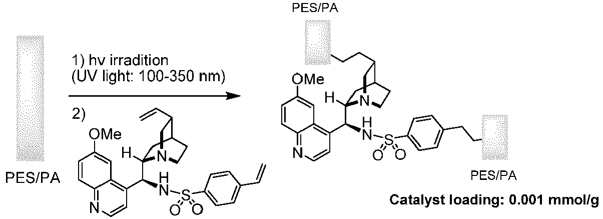

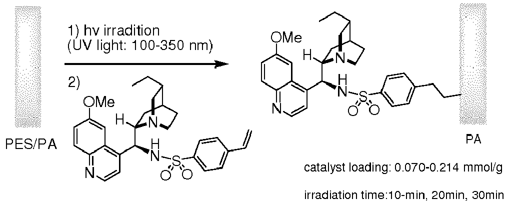

| Entry | Irradiation time | Concentration of monomer | Catalyst loading on textile |
|---|---|---|---|
| | [min] | [mmol/g] | [mmol/g] |
| 1 | 10 | 0,05 | 0,166 |
| 2 | 10 | 0,1 | 0,188 |
| 3 | 20 | 0,1 | 0,214 |
| 4 | 30 | 0,1 | 0,194 |
| 5 | 30 | 0,25 | 0,070 |

### Example 3

The photochemical immobilization of a quinine-based sulfonamide catalyst on a polymeric support such PES/PA is generally shown in the following scheme:

The catalytic activity and enantioselectivity are as follows:

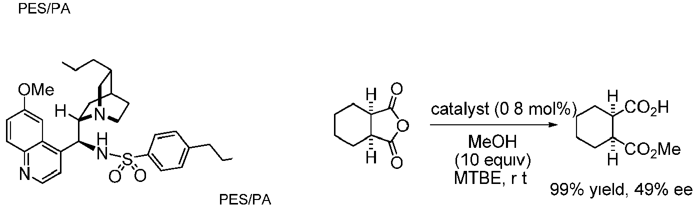

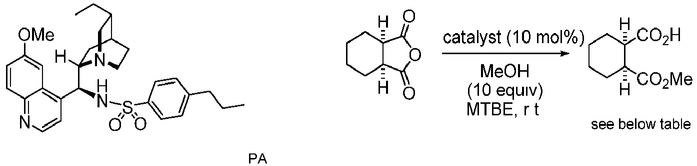

| Entry | Irradiation time | Concentration of monomer | Catalyst loading on textile | ee (%) |
|---|---|---|---|---|
| | [min] | [mmol/g] | [mmol/g] | |
| 1 | 10 | 0,05 | 0,166 | 13% |
| 2 | 10 | 0,1 | 0,188 | 25% |
| 3 | 20 | 0,1 | 0,214 | 38% |
| 4 | 30 | 0,1 | 0,194 | 32% |
| 5 | 30 | 0,25 | 0,070 | 54% |

### Example 4

The photochemical immobilization of a quinine-based sulfonamide catalyst on a support material or, more specifically, a textile material selected from woven or non-woven polyester, polyamide (or Nylon) and cotton is generally shown in the following scheme:

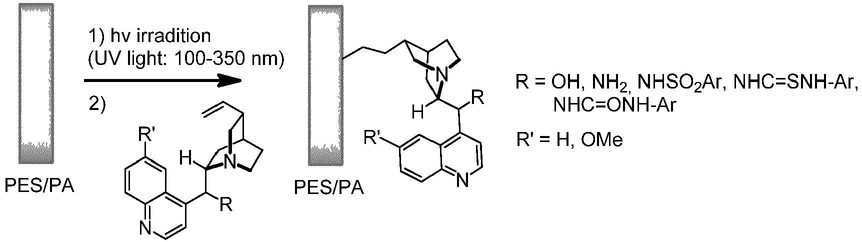

The photochemical immobilization of quinine-based sulfonamide catalysts on a polymeric support such as PA is generally shown for different loads in the following schemes:

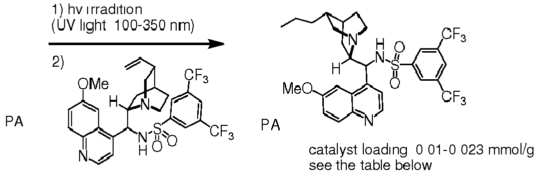

| Entry | Concentration of monomer | Irradiation time | Crosslinker | Amount of crosslinker mmol/g | Catalyst loading on textile |
|---|---|---|---|---|---|
| 1 | 0,025 | 2 x 5min | | | 0.0122 |
| 2 | 0,1 | 2 x 5min | | | 0.0154 |
| 3 | 0,3 | 2 x 5min | peta | 0,17 | 0.0228 |
| 4 | 0,3 | 2 x 5min | | | 0.0151 |
| 5 | 0,3 | 2 x l0min | | | 0.0116 |

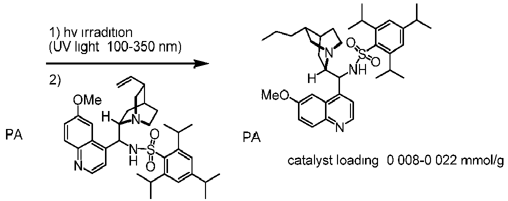

### Example 5

For a Quinine-thiourea catalyst on polyamide, the catalytic activity and enantioselectivitiy as well as recyclability of the textile catalyst have been evaluated as follows:

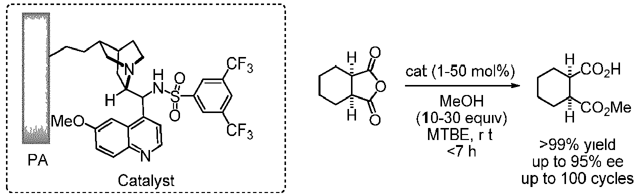

The catalytic reaction was conducted in 15 mL glass vial equipped with textile catalyst and magnetic stirring bar. Cyclohexanedicarboxylic anhydride was added and dissolved with MTBE (> 10 mL). Then methanol (2-10 equiv) was added and the reaction mixture was stirred until the starting material consumed completely. Then the reaction vial was washed with MTBE (10 mL) 4 times and the combined organic layer was evaporated and dried under vacuo to afford the desired product (>99% purity by ¹H NMR) and then converted to the diastereomeric mixture to determine the ee values.

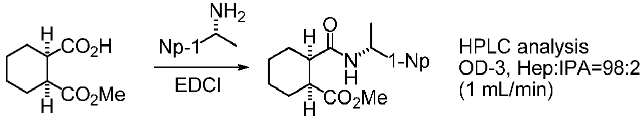

The recycling experiment was conducted using the same reaction vial by adding more substrates and solvent without further purification. The recyclability is shown in Figure 2 showing that the inventive catalyst can be recycled for more than 100 times without a loss of activity.

The substrate scope of the immobilized QN-sulfonamide catalyst is shown in the following scheme:

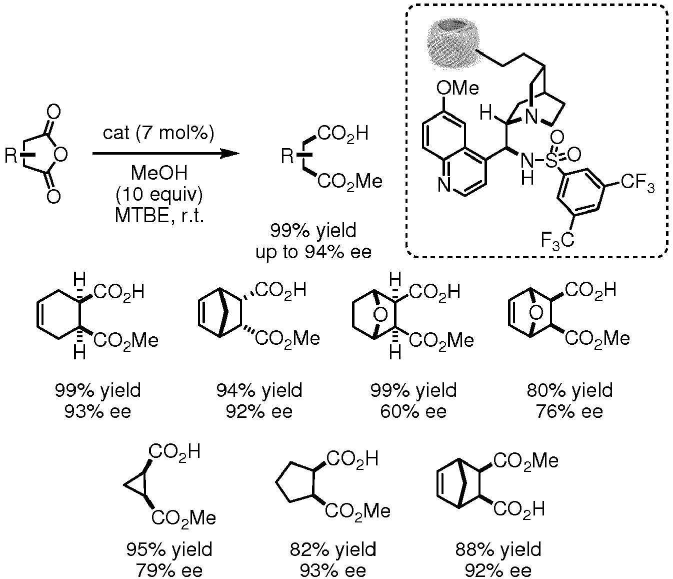

### Example 6

The immobilization of Quinine-thiourea catalyst on polyamide yielded catalysts with differing loadings 0,007 to 0,012 mmol/g support as follows:

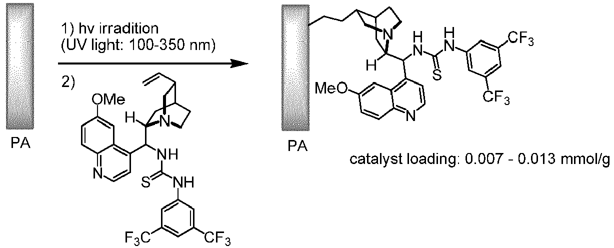

The application of quinine-thiourea textile catalyst revealed:

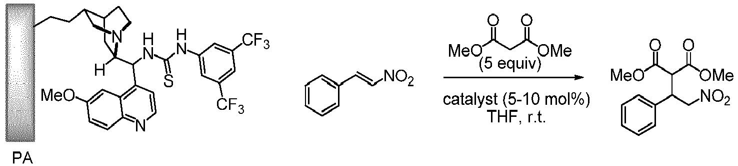

### Example 7

The synthesis and immobilization of phosphoric acid catalyst is exemplified for one compound in the following scheme:

### A. Synthesis of allyl-substituted TRIP-phosphate

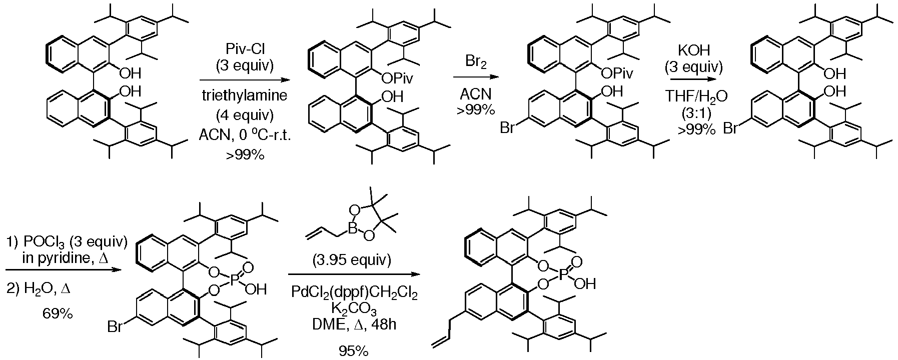

### B. Immobilization of allyl substituted phosphoric acid

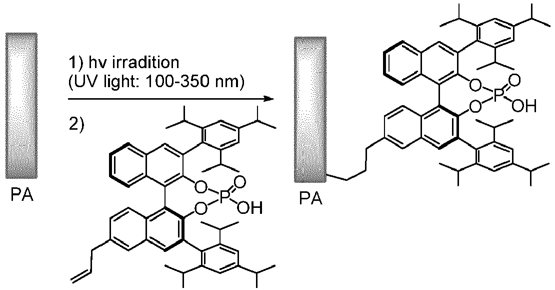

### Example 8

The synthesis and immobilization of imidodiphosphoric acid catalyst is exemplified for one compound in the following scheme:

### A. Synthesis of allyl substituted imidodiphosphoric acid

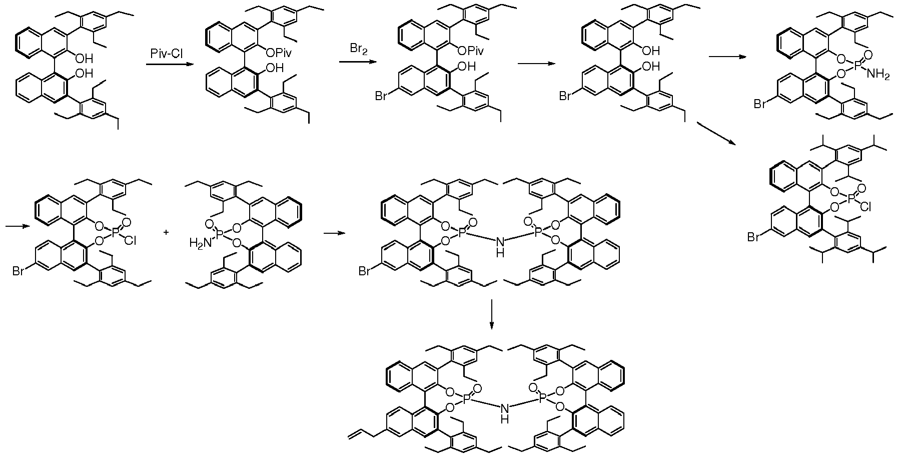

### B. Immobilization of allyl substituted imidodiphosphoric acid

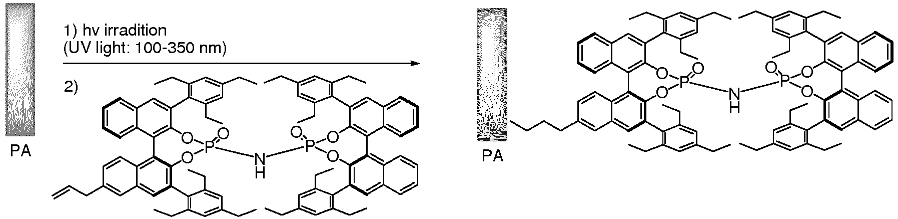

### Example 9

The synthesis and immobilization of primary- and secondary amine catalysts is exemplified for three compounds in the following schemes:

### A. Synthesis and immobilization of Quinine-derived primary amine catalyst

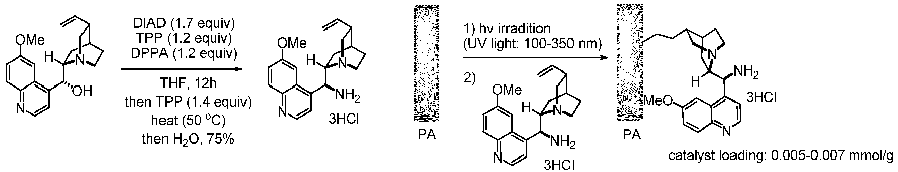

### B. Synthesis and immobilization of proline catalyst ant its use

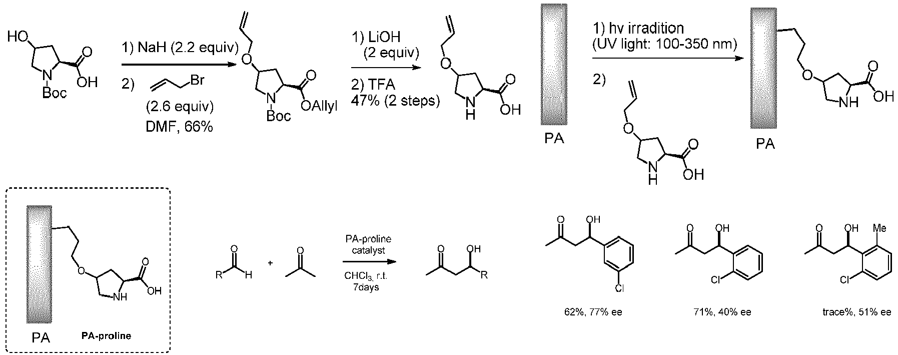

### C. Synthesis and immobilization of phenylalanine-derived secondary amine

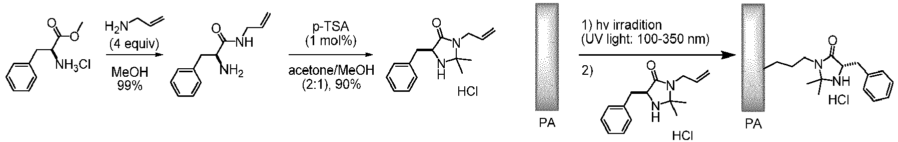

### Example 10

The synthesis and immobilization of a nucleophilic DMAP-type catalyst is exemplified for one compound in the following scheme:

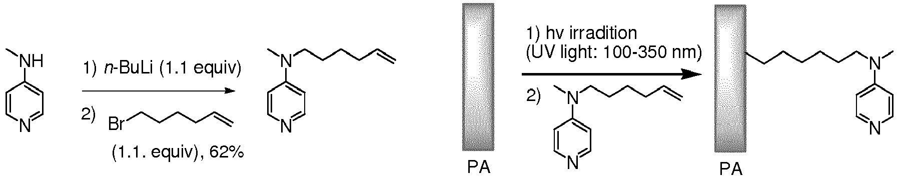

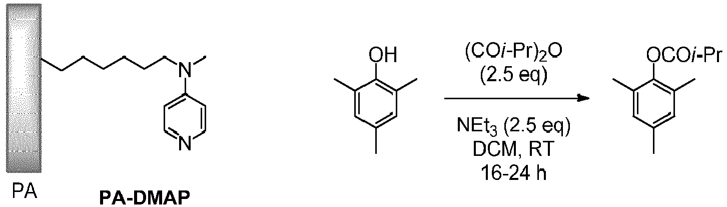

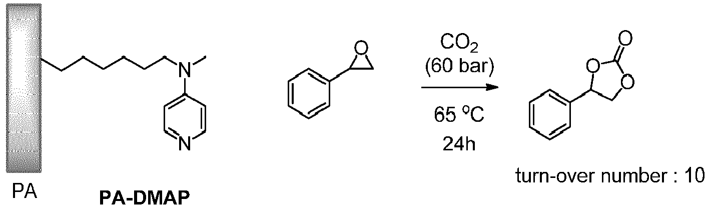

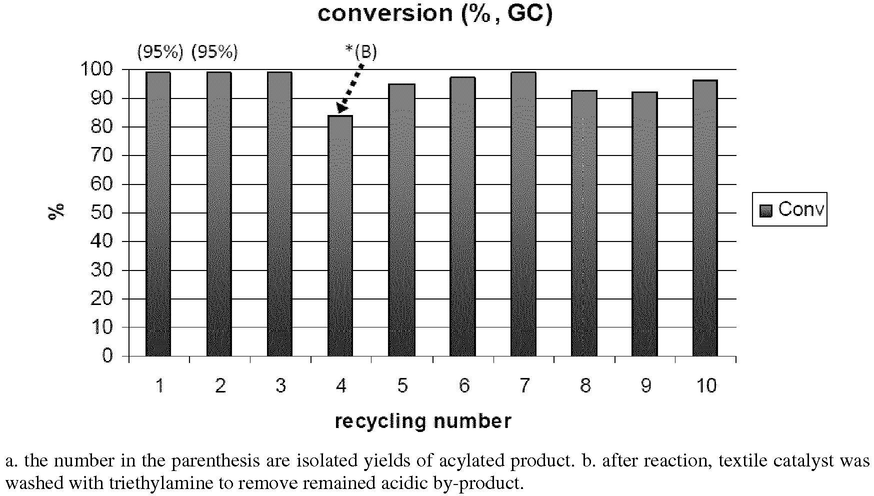

### Example 11

The synthesis and immobilization of a TEMPO-like catalyst is exemplified in the following scheme:

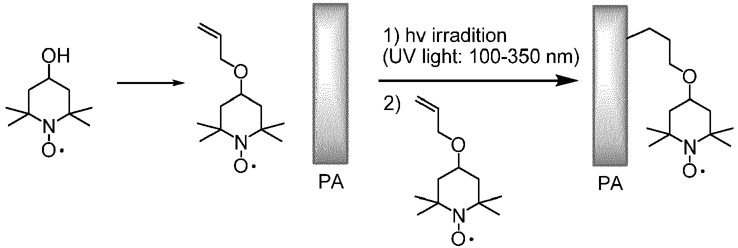

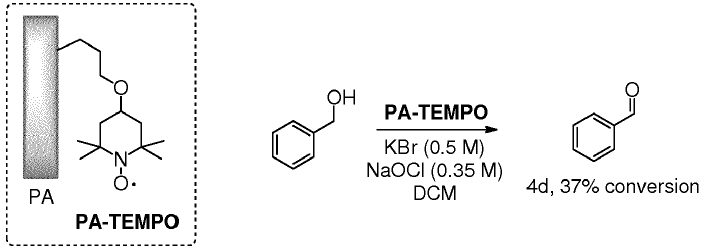

### Example 12

The synthesis and immobilization of a phase-transfer catalyst is exemplified in the following scheme

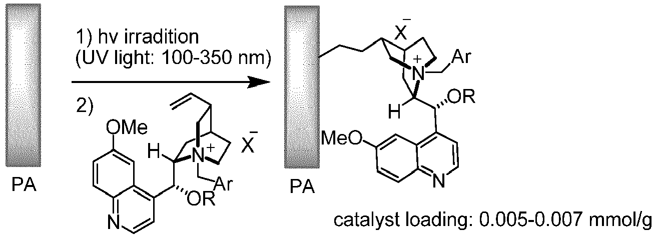

## Claims

1. A process for immobilization of at least one organic catalyst on a polymeric support, wherein said organic catalyst is permanently bonded to the polymeric support by a chemical interaction between at least one functional group on either of the organic catalyst and the polymeric support.

2. The process according to claim 1 wherein the chemical interaction is a chemical bonding via a ionic or radically induced reaction.

3. The process according to claim 1 or 2 wherein the chemical interaction is induced by a photochemical process.

4. The process according to any one of preceding claims wherein the organic catalyst is a heterogeneous organic catalyst, preferably selected from any of cinchona alkaloid-based bifunctional catalysts, BINOL-based phosphoric acids, BINOL-based imidodiphosphoric acids, secondary- and primary amine catalysts, nitrogen-based nucleophilic catalysts, TEMPO as an organic oxidant, phase-transfer catalysts as well as imidodiphosphoric acids as disclosed in EP application No.12150663.8.

5. The process according to any one of preceding claims wherein the polymeric support is selected from the group of natural or synthetic textile materials or mixtures thereof, preferably selected from polyesters, polyamides, polyacrylates, polyolefins, cotton, rayon and wool.

6. The process according to any one of preceding claims 1 to 5, wherein the organic catalyst is immobilized on the polymeric support via a photochemical reaction between at least one functional crosslinking olefinic group on the organic catalyst with the polymeric support, either directly or via a linker molecule having at least two vinylic or allylic olefinic groups such as PETA.

7. The process of claim 6 wherein the functional crosslinking olefinic group is a vinyl or allyl group attached to the organic catalyst.

8. The process of any one of claims 6 or 7 wherein the photochemical reaction is induced by irradiation with UV light.

9. The process of claim 8, wherein the irradiation with UV light of a wavelength in the range of 100 to 350 nm, preferably with a wavelength of 222 nm is used.

10. The process of any one of the claims 6 to 9, wherein the polymeric support is treated with a solution of the organic catalyst having at least one functional crosslinking olefinic group in a photochemically inert organic solvent, the obtained impregnated polymeric support is irradiated with UV light and the obtained polymeric support having the organic catalyst covalently fixed thereon is recovered and optionally washed with an organic solvent.

11. Organic catalyst on a polymeric support, obtainable by a process according to any preceding claim.

12. Use of the organic catalyst on a polymeric support according to claim 11 as catalyst in chemical reactions.
